(19) Europäisches Patentamt
European Patent Office
Office européen des brevets

(11) Veröffentlichungsnummer: **0 063 381**
**A1**

# (12) EUROPÄISCHE PATENTANMELDUNG

(21) Anmeldenummer: **82103302.4**

(22) Anmeldetag: **20.04.82**

(51) Int. Cl.$^3$: **C 07 D 487/04**
A 61 K 31/505
//(C07D487/04, 239/00, 231/00)

(30) Priorität: **22.04.81 CH 2621/81**
**26.03.82 CH 1872/82**

(43) Veröffentlichungstag der Anmeldung:
**27.10.82 Patentblatt 82/43**

(84) Benannte Vertragsstaaten:
**IT**

(71) Anmelder: **Byk Gulden Lomberg Chemische Fabrik GmbH**
**Byk-Gulden-Strasse 2**
**D-7750 Konstanz(DE)**

(72) Erfinder: **Gauri, Kailash Kumar, Prof. Dr.**
**Zur Waldburg 13**
**D-2359 Lentföhrden(DE)**

(72) Erfinder: **Erbler, Hans, Prof. Dr.**
**Zum Guckenbühl 7**
**D-7750 Konstanz 16(DE)**

(72) Erfinder: **Eltze, Manfrid, Dr.**
**Schützenstrasse 20**
**D-7750 Konstanz(DE)**

(54) **Neue Pyrazolo(3,4-d)pyrimidine, Verfahren zu deren Herstellung und sie enthaltende Arzneimittel.**

(57) Verbindungen der allgemeinen Formel I

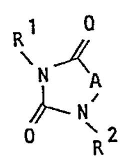

worin $R^1$ und $R^2$, die gleich oder verschieden sind, für Wasserstoff oder die Reste $C_1$-$C_8$-Alkyl, $C_2$-$C_8$-Alkenyl, $C_4$-$C_8$-Alkylcarbonylalkyl, $C_3$-$C_8$-Alkylthioalkyl und $C_3$-$C_8$-Alkoxyalkyl, die durch Halogen, Hydroxy oder den Di($C_1$-$C_2$-Alkyl)aminorest substituiert sein können, $R^1$ und $R^2$ jedoch nicht gleichzeitig Wasserstoff sein können, und A für eine Gruppe

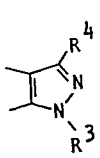

oder

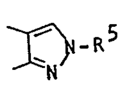

,

wobei $R^3$ Wasserstoff oder $C_1$-$C_4$-Alkyl, $R^4$ Wasserstoff, $C_1$-$C_4$-Alkyl oder Phenyl, das bis zu dreifach $C_1$-$C_2$-alkoxysubstituiert sein kann, $R^5$ Wasserstoff, $C_1$-$C_6$-Alkyl, Hydroxy-$C_1$-$C_3$-alkyl, Di($C_1$-$C_2$-alkyl)-amino-$C_1$-$C_4$-alkyl, $C_2$-$C_6$-Alkenyl, Phenyl, Phenyl-$C_1$-$C_2$-alkyl oder Benzoyl, wobei die Phenylringe der letztgenannten drei Reste bis zu dreifach $C_1$-$C_2$-alkoxysubstituiert sein können, $C_1$-$C_3$-Alkylthio-$C_2$-$C_4$-alkyl oder Hydroxy-, Amino oder Sulfo-$C_1$-$C_3$-alkylthio-$C_2$-$C_4$-alkyl bedeuten, stehen, wobei $R^1$, $R^2$ und $R^5$ nicht gleichzeitig Ethyl bedeuten, $R^1$ und $R^2$ nicht beide Methyl bedeuten, wenn $R^3$ oder $R^5$ Butyl oder Di($C_1$-$C_2$-alkyl)aminoethyl bedeuten, und die Summe der Kohlenstoff-, Sauerstoff-, Stickstoff- und Schwefelatome der an Ringstickstoffatome gebundenen Ketten zusammen mindestens 6 beträgt, oder mindestens 5 beträgt, wenn $R^2$ Isobutyl bedeutet, weisen eine starke bronchospasmolytische, phosphodiesterasehemmende und retinotrope Wirkung auf. Sie verbessern die rheologischen Eigenschaften des Blutes und die periphere Durchblutung. Sie eignen sich daher zur Herstellung von Arzneimitteln. Verfahren zur Herstellung der neuen Verbindungen werden angegeben.

EP 0 063 381 A1

Croydon Printing Company Ltd.

# Neue Pyrazolo[3,4-d]pyrimidine, Verfahren zu deren Herstellung und sie enthaltende Arzneimittel

## Technisches Gebiet

Die Erfindung bezieht sich auf neue Pyrazolo[3,4-d]pyrimidine, diese enthaltende Arzneimittel und Verfahren zu deren Herstellung.

## Stand der Technik

Aus der DE-PS 11 06 330 sind 5-Alkyl-4,5,6,7-tetrahydro-4,6-dioxo-1H-pyrazolo[3,4-d]pyrimidine mit koffeinartiger Wirkung bekannt. In der DE-AS 11 86 466 wird ein Verfahren zur Herstellung von bis zu dreifach N-substituierten 4,5,6,7-Tetrahydro-4,6-dioxo-1H-pyrazolo-[3,4-d]pyrimidinen angegeben. Über die Verfahrensprodukte wird ganz allgemein gesagt, sie würden wertvolle pharmakologische Eigenschaften besitzen. In J.Heterocycl.Chem. 15,359(1978) wird die Herstellung von 2-Alkyl-4,5,6,7-tetrahydro-5,7-dimethyl-4,6-dioxo-2H-pyrazolo-[3,4-d]pyrimidinen, unter anderem von 2-n-Butyl-4,5,6,7-tetrahydro-5,7-dimethyl-4,6-dioxo-2H-pyrazolo[3,4-d]pyrimidin, beschrieben. In der AT-PS 204.557 und der US-PS 3.098.075 werden die Herstellung von unter anderem durch Aminoalkylreste N-substituierte 4,5,6,7-Tetrahydro-4,6-dioxo-1H-pyrazolo[3,4-d]pyrimidinen angegeben. In der US-PS 3,113,944 werden 2,4,6-Trialkyl-4,5,6,7-tetrahydro-5,7-dioxo-2H-pyrazolo[4,3-d]pyrimidine offenbart.

## Darstellung der Erfindung

Überraschend wurde nun festgestellt, daß die neuen Verbindungen der allgemeinen Formel I

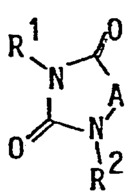

(I),

worin

$R^1$ und $R^2$, die gleich oder verschieden sind, für Wasserstoff oder die Reste $C_1$-$C_8$-Alkyl, $C_2$-$C_8$-Alkenyl, $C_4$-$C_8$-Alkylcarbonylalkyl, $C_3$-$C_8$-Alkylthioalkyl und $C_3$-$C_8$-Alkoxyalkyl, die durch Halogen, Hydroxy oder den Di($C_1$-$C_2$-Alkyl)aminorest substituiert sein können, $R^1$ und $R^2$ jedoch nicht gleichzeitig Wasserstoff sein können, und

A für eine Gruppe 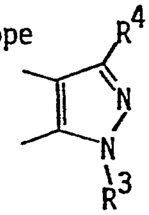 oder 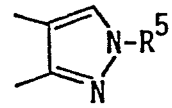,

wobei

$R^3$ Wasserstoff oder $C_1$-$C_4$-Alkyl,

$R^4$ Wasserstoff, $C_1$-$C_4$-Alkyl oder Phenyl, das bis zu dreifach $C_1$-$C_2$-alkoxysubstituiert sein kann,

$R^5$ Wasserstoff, $C_1$-$C_6$-Alkyl, Hydroxy-$C_1$-$C_3$-alkyl, Di($C_1$-$C_2$-alkyl)amino-$C_1$-$C_4$-alkyl, $C_2$-$C_6$-Alkenyl, Phenyl, Phenyl-$C_1$-$C_2$-Alkyl oder Benzoyl, wobei die Phenylringe der letztgenannten drei Reste bis zu dreifach $C_1$-$C_2$-alkoxysubstituiert sein können, $C_1$-$C_3$-Alkylthio-$C_2$-$C_4$-alkyl oder Hydroxy-, Amino- oder Sulfo-$C_1$-$C_3$-alkylthio-$C_2$-$C_4$-alkyl bedeuten,

stehen, wobei $R^1$, $R^2$ und $R^5$ nicht gleichzeitig Ethyl bedeuten, $R^1$ und $R^2$ nicht beide Methyl bedeuten, wenn $R^3$ oder $R^5$ Butyl oder Di($C_1$-$C_2$-alkyl)aminoethyl bedeuten, und die Summe der Kohlenstoff-, Sauerstoff-, Stickstoff- und Schwefelatome der an Ringstickstoffatome gebundenen Ketten zusammen mindestens 6 beträgt, oder mindestens 5 beträgt, wenn $R^2$ Isobutyl bedeutet, eine starke bronchospasmolytische, phosphodiesterasehemmende und retinotrope Wirkung aufweisen. Außerdem verbessern sie die rheologischen Eigenschaften des Blutes und damit die periphere Durchblutung. Sie eignen sich daher beispielsweise zur Behandlung obstruktiver Atemwegserkrankungen, cerebraler und peripherer Durchblutungsstörungen, von Durchblutungsstörungen der Netz- und Aderhaut, Altersschwerhörigkeit, Ohrensausen und otogenen Schwindels. Die erfindungsgemäßen Verbindungen wirken nicht mutagen.

Gegenstand der Erfindung sind daher Pyrazolo[3,4-d]pyrimidine der vorstehenden allgemeinen Formel I, worin A, $R^1$ und $R^2$ die angegebenen Bedeutungen haben, deren Anwendung zur Behandlung der angegebenen Gesundheitsstörungen und zur Herstellung von Arzneimitteln, insbesondere solcher zur Behandlung der angegebenen Gesundheitsstörungen und diese Verbindungen enthaltende Arzneimittel, gewünschtenfalls in Verbindung mit geeigneten Träger- bzw. Hilfsstoffen.

Bevorzugt sind Verbindungen der allgemeinen Formel I',

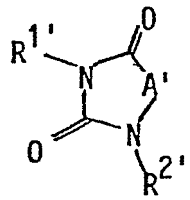 (I'),

worin

$R^{1'}$ und $R^{2'}$, die gleich oder verschieden sind, für Wasserstoff oder die Reste $C_1$-$C_8$-Alkyl, $C_2$-$C_4$-Alkenyl, $C_5$-$C_7$-Alkylcarbonylalkyl, $C_5$-$C_7$-Alkylthioalkyl und $C_3$-$C_7$-Alkoxyalkyl, die durch Hydroxy oder den Dimethylaminorest substituiert sein können, $R^{1'}$ und $R^{2'}$ jedoch nicht gleichzeitig Wasserstoff sein können, und

A' für eine Gruppe 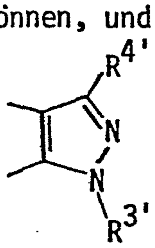 oder 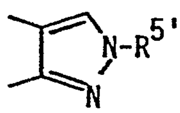,

wobei

$R^{3'}$ Wasserstoff oder $C_1$-$C_3$-Alkyl

$R^{4'}$ Wasserstoff, $C_1$-$C_3$-Alkyl oder Phenyl, das bis zu dreifach methoxysubstituiert sein kann, und

$R^{5'}$ Wasserstoff, $C_1$-$C_6$-Alkyl, Hydroxy-$C_1$-$C_3$-alkyl, Di($C_1$-$C_2$-alkyl)aminoethyl, $C_2$-$C_3$-Alkenyl, Phenyl, Benzyl, Benzoyl, wobei die Phenylringe der letztgenannten drei Reste durch bis zu drei Methoxygruppen substituiert sein können, oder Hydroxy-$C_1$-$C_2$-alkylthio-$C_2$-$C_3$-alkyl bedeuten,

stehen, wobei $R^{1'}$, $R^{2'}$ und $R^{5'}$ nicht gleichzeitig Ethyl bedeuten, $R^{1'}$ und $R^{2'}$ nicht beide Methyl bedeuten, wenn $R^{5'}$ Butyl oder Di($C_1$-$C_2$-alkyl)aminoethyl bedeutet, und die Summe der Kohlenstoff-,

Sauerstoff-, Stickstoff- und Schwefelatome der an Ringstickstoffatome gebundenen Ketten zusammen mindestens 6 beträgt, oder mindestens 5 beträgt, wenn $R^{2'}$ Isobutyl bedeutet, sowie diese Verbindungen enthaltende Arzneimittel.

Besonders bevorzugt sind Verbindungen der allgemeinen Formel I",

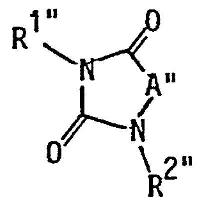

(I"),

worin

$R^{1"}$ und $R^{2"}$, die gleich oder verschieden sind, für Wasserstoff, $C_1$-$C_6$-Alkyl, 2-Hydroxyethyl, Allyl, 5-Oxo-n-hexyl, Hydroxyethoxymethyl, Hydroxyethoxy-n-propyl, Hydroxyethylthio-n-propyl oder 2-(Dimethylamino)ethyl, $R^{1"}$ und $R^{2"}$ jedoch nicht gleichzeitig Wasserstoff sein können,

und A" für eine Gruppe

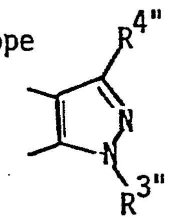

oder

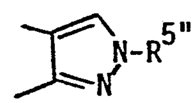

wobei

$R^{3"}$ Wasserstoff, Methyl oder Ethyl,

$R^{4"}$ Wasserstoff oder Phenyl und

$R^{5"}$ Wasserstoff, $C_1$-$C_6$-Alkyl, 2-Hydroxyethyl, Allyl, Trimethoxyphenyl, Trimethoxybenzoyl oder Hydroxyethylthio-n-propyl bedeuten,

stehen, wobei $R^{1"}$, $R^{2"}$ und $R^{5"}$ nicht gleichzeitig Ethyl bedeuten, $R^{1"}$ und $R^{2"}$ nicht beide Methyl bedeuten, wenn $R^{5"}$ Butyl bedeutet, und die Summe der Kohlenstoff-, Sauerstoff-, Stickstoff- und Schwefelatome der an Ringstickstoffatome gebundenen Ketten zusammen mindestens 6 beträgt, oder mindestens 5 beträgt, wenn $R^{2"}$ Isobutyl bedeutet, und diese Verbindungen enthaltende Arzneimittel.

Ganz besonders bevorzugt sind Verbindungen der allgemeinen Formel I''',

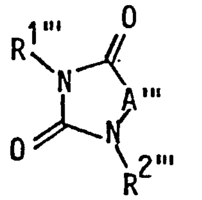

(I'''),

worin einer der Reste $R^{1'''}$ und $R^{2'''}$ für Wasserstoff, Methyl oder Ethyl, der andere für Ethyl, Butyl, Hexyl, 5-Oxo-n-hexyl oder 2-(Dimethylamino)ethyl, und

A''' für eine Gruppe

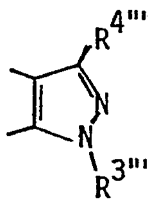

oder

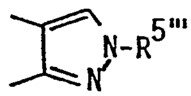

wobei

$R^{3'''}$ Wasserstoff oder Methyl,

$R^{4'''}$ Wasserstoff und

$R^{5'''}$ $C_1$-$C_6$-Alkyl, 2-Hydroxyethyl und Trimethoxybenzoyl bedeuten,

stehen, wobei die Summe der Kohlenstoff-, Sauerstoff- und Stickstoffatome der an Ringstickstoffatome gebundenen Ketten zusammen mindestens 6 beträgt, oder mindestens 5 beträgt, wenn $R^{2'''}$ Isobutyl bedeutet, und diese Verbindungen enthaltende Arzneimittel.

Ein Butylrest ist ein n-, i-, sek.- oder tert.-Butylrest.

Ein Rest $C_4$-$C_8$-Alkylcarbonylalkyl ist beispielsweise ein 3-Oxo-n-butyl-, 3-Oxo-n-pentyl-, 4-Oxo-n-pentyl-, 3-Oxo-n-hexyl-, 5-Oxo-n-hexyl-, 6-Oxo-n-heptyl- oder 7-Oxo-n-octyl-Rest, vorzugsweise ein 5-Oxo-n-hexyl-Rest.

Ein Rest $C_3$-$C_8$-Alkylthioalkyl ist beispielsweise ein Methylthioethyl-, Methylthiopropyl-, Ethylthioethyl-, n-Propylthioethyl-, Ethylthio-n-butyl-, Isopropylthio-n-butyl- oder Isobutylthio-n-butyl-Rest, vorzugsweise ein Ethylthiopropyl-Rest.

Ein Rest $C_3$-$C_8$-Alkoxyalkyl ist beispielsweise ein Methoxyethyl-, Methoxypropyl-, Ethoxyethyl-, n-Propoxyethyl-, Ethoxy-n-butyl-, Isopropoxy-m-butyl- oder Isobutoxy-n-butyl-Rest, vorzugsweise ein Ethoxypropyl-Rest.

Bei den Resten $C_4$-$C_8$- bzw. $C_5$-$C_7$-Alkylcarbonylalkyl, $C_3$-$C_8$- bzw. $C_5$-$C_7$-Alkylthioalkyl und $C_3$-$C_8$- bzw. $C_3$-$C_7$-Alkoxyalkyl bezieht sich die angegebene Zahl von C-Atomen jeweils auf alle C-Atome des Restes.

Ein durch Halogen oder Hydroxy substituierter Rest $C_4$-$C_8$-Alkylcarbonylalkyl, $C_3$-$C_8$-Alkylthioalkyl oder $C_3$-$C_8$-Alkoxyalkyl ist beispielsweise ein 5-Hydroxy-3-oxo-n-pentyl-, Hydroxyethylthioethyl-, Hydroxyethoxyethyl-, Hydroxyethoxy-n-propyl-, Hydroxyethylthio-n-propyl-, 5-Chlor-3-oxo-n-pentyl-, (2-Chlorethyl)-thioethyl-, (2-Chlorethoxy)-ethyl-, (2-Chlorethoxy)-n-propyl- oder (2-Chlorethyl)-thio-n-propyl-Rest, vorzugsweise ein (2-Hydroxy)-thio-n-propyl-, (2-Hydroxyethoxy)-n-propyl- oder (2-Hydroxyethoxy)-methyl-Rest.

Ein Rest Amino- oder Sulfo-$C_1$-$C_3$-alkylthio-$C_2$-$C_4$-alkyl ist beispielsweise ein (2-Aminoethyl)-thioethyl-, (2-Sulfoethyl)-thiopropyl-, (2-Aminopropyl)-thio-n-butyl- oder (3-Sulfopropyl)-thio-n-propyl-Rest.

Zur Bestimmung der Summe der Kohlenstoff-, Sauerstoff-, Stickstoff- und Schwefelatome der an Ringstickstoffatome gebundenen Ketten werden nur die direkt an die Stickstoffatome in Position 1, 2, 5 oder 7 des Pyrazolo[3,4-d]pyrimidingerüsts gebundenen aliphatischen Teile der Reste berücksichtigt. So werden bei der Summenbildung aus den einzelnen Ketten beispielsweise Methyl als 1, Hydroxyethyl als 3, Isobutyl als 4, Benzyl als 1, Benzoyl ebenso wie Trimethoxybenzoyl als 2 und 5-Oxo-n-hexyl als 7 berücksichtigt.

Als Beispiele für besonders bevorzugte Verbindungen seien genannt:

7-n-Hexyl-4,5,6,7-tetrahydro-1,5-dimethyl-4,6-dioxo-1H-pyrazolo[3,4-d]-pyrimidin (Schmp. 111°C);

5-n-Hexyl-4,5,6,7-tetrahydro-1,7-dimethyl-4,6-dioxo-1H-pyrazolo[3,4-d]-pyrimidin;

7-i-Butyl-4,5,6,7-tetrahydro-1,5-dimethyl-4,6-dioxo-1H-pyrazolo[3,4-d]-pyrimidin;

5-n-Hexyl-4,5,6,7-tetrahydro-2-(3,4,5-trimethoxyphenyl)-4,6-dioxo-2H-pyrazolo[3,4-d]pyrimidin ;

5-n-Hexyl-4,5,6,7-tetrahydro-2-(3,4,5-trimethoxybenzoyl)-7-methyl-4,6-dioxo-2H-pyrazolo[3,4-d]pyrimidin (Schmp. 150°C);

2-Ethyl-5-n-hexyl-4,5,6,7-tetrahydro-7-methyl-4,6-dioxo-2H-pyrazolo-[3,4-d]pyrimidin (Schmp. 110°C);

2-n-Butyl-7-n-hexyl-4,5,6,7-tetrahydro-5-methyl-4,6-dioxo-2H-pyrazolo-[3,4-d]pyrimidin (Schmp. 67°C);

4,5,6,7-Tetrahydro-7-(2-hydroxyethoxymethyl)-1-methyl-4,6-dioxo-1H-pyrazolo[3,4-d]pyrimidin;

4,5,6,7-Tetrahydro-5-methyl-4,6-dioxo-7-(5-oxo-n-hexyl)-2H-pyrazolo-[3,4-d]pyrimidin;

4,5,6,7-Tetrahydro-7-(2-hydroxyethoxymethyl)-5-methyl-4,6-dioxo-1H-pyrazolo[3,4-d]pyrimidin;

5-i-Butyl-4,5,6,7-tetrahydro-2-(3,4,5-trimethoxybenzoyl)-7-methyl-4,6-dioxo-2H-pyrazolo[3,4-d]pyrimidin;

4,5,6,7-Tetrahydro-1-methyl-4,6-dioxo-5-(5-oxo-n-hexyl)-3-n-propyl-1H-pyrazolo[3,4-d]pyrimidin;

1-Ethyl-4,5,6,7-tetrahydro-5-[3-(2-hydroxyethylthio)-n-propyl]-7-methyl-4,6-dioxo-1H-pyrazolo[3,4-d]pyrimidin;

5-n-Hexyl-4,5,6,7-tetrahydro-2,7-dimethyl-4,6-dioxo-2H-pyrazolo-[3,4-d]pyrimidin (Schmp. 73-75°C);

2-n-Butyl-5-n-hexyl-4,5,6,7-tetrahydro-7-methyl-4,6-dioxo-2H-pyrazolo[3,4-d]pyrimidin (Schmp. 88-90°C);

2,5-Di-n-hexyl-4,5,6,7-tetrahydro-7-methyl-4,6-dioxo-2H-pyrazolo-[3,4-d]pyrimidin (Schmp. 80-81°C);

7-n-Hexyl-4,5,6,7-tetrahydro-5-methyl-4,6-dioxo-2H-pyrazolo[3,4-d]-pyrimidin (Schmp. 159-161°C);

7-n-Hexyl-4,5,6,7-tetrahydro-2,5-dimethyl-4,6-dioxo-2H-pyrazolo[3,4-d]-pyrimidin (Schmp. 109-110°C);

2,7-Di-n-hexyl-4,5,6,7-tetrahydro-5-methyl-4,6-dioxo-2H-pyrazolo[3,4-d]-pyrimidin (Schmp. 96-97°C);

4,5,6,7-Tetrahydro-7-[3-(2-hydroxyethylthio)-n-propyl]-5-methyl-4,6-dioxo-2H-pyrazolo[3,4-d]pyrimidin (Schmp. 273°C);

4,5,6,7-Tetrahydro-7-(2-hydroxyethoxy)-2,5-dimethyl-4,6-dioxo-2H-pyrazolo[3,4-d]pyrimidin;

4,5,6,7-Tetrahydro-2-methyl-4,6-dioxo-7-(5-oxo-n-hexyl)-2H-pyrazolo-[3,4-d]pyrimidin;

7-Butyl-4,5,6,7-tetrahydro-4,6-dioxo-2-(2-phenylethyl)-2H-pyrazolo-[3,4-d]pyrimidin;

7-Allyl-2-n-hexyl-4,5,6,7-tetrahydro-5-methyl-4,6-dioxo-2H-pyrazolo-[3,4-d]pyrimidin;

5-(2-Chlorethyl)-7-n-hexyl-4,5,6,7-tetrahydro-2-methyl-4,6-dioxo-2H-pyrazolo[3,4-d]pyrimidin;

1-i-Butyl-4,5,6,7-tetrahydro-5,7-dimethyl-4,6-dioxo-3-phenyl-1H-pyrazolo[3,4-d]pyrimidin;

4,5,6,7-Tetrahydro-1-(4-methoxyphenyl)-4,6-dioxo-5-(3-oxobutyl)-1H-pyrazolo[3,4-d]pyrimidin;

4,5,6,7-Tetrahydro-3,5-dimethyl-7-(2-methylbutyl)-4,6-dioxo-1H-pyrazolo-[3,4-d]pyrimidin;

4,5,6,7-Tetrahydro-1,5-dimethyl-7-(2-methylbutyl)-4,6-dioxo-1H-pyrazolo-[3,4-d]pyrimidin;

5-n-Hexyl-4,5,6,7-tetrahydro-7-methyl-4,6-dioxo-3-phenyl-1H-pyrazolo-[3,4-d]pyrimidin (Schmp. 196-198°C);

7-n-Hexyl-4,5,6,7-tetrahydro-5-methyl-4,6-dioxo-2-n-propyl-2H-pyrazolo-[3,4-d]pyrimidin;

4,5,6,7-Tetrahydro-1,5-dimethyl-4,6-dioxo-7-(5-oxo-n-hexyl)-1H-pyrazolo-[3,4-d]pyrimidin;

2-Ethyl-7-n-hexyl-4,5,6,7-tetrahydro-5-methyl-4,6-dioxo-2H-pyrazolo-[3,4-d]pyrimidin (Schmp. 96-97°C);

5-n-Hexyl-4,5,6,7-tetrahydro-7-methyl-4,6-dioxo-1H-pyrazolo[3,4-d]pyrimidin (Schmp. 138°C);

3-Ethyl-5-n-hexyl-4,5,6,7-tetrahydro-1,7-dimethyl-4,6-dioxo-1H-pyrazolo-[3,4-d]pyrimidin;

5-n-Hexyl-4,5,6,7-tetrahydro-3-(3,4,5-trimethoxyphenyl)-1,7-dimethyl-4,6-dioxo-1H-pyrazolo[3,4-d]pyrimidin;

7-i-Butyl-4,5,6,7-tetrahydro-5-methyl-4,6-dioxo-1H-pyrazolo[3,4-d]pyrimidin (Schmp. 204°C);

4,5,6,7-Tetrahydro-2-(2-hydroxyethyl)-4,6-dioxo-5-(3-oxobutyl)-2H-pyrazolo[3,4-d]pyrimidin;

7-i-Butyl-4,5,6,7-tetrahydro-2-(2-hydroxyethyl)-5-methyl-4,6-dioxo-2H-pyrazolo[3,4-d]pyrimidin;

4,5,6,7-Tetrahydro-1,5-dimethyl-7-(2-dimethylaminoethyl)-4,6-dioxo-1H-pyrazolo[3,4-d]pyrimidin;

7-i-Butyl-4,5,6,7-tetrahydro-5-methyl-2-(2-dimethylaminoethyl)-4,6-dioxo-2H-pyrazolo[3,4-d]pyrimidin;

7-Ethyl-4,5,6,7-tetrahydro-1-methyl-4,6-dioxo-5-(5-oxo-n-hexyl)-1H-pyrazolo[3,4-d]pyrimidin,

7-i-Butyl-4,5,6,7-tetrahydro-2-(2-hydroxyethyl)-5-methyl-4,6-dioxo-2H-pyrazolo[3,4-d]pyrimidin (Schmp. 157,5-158,5°C);
7-Ethyl-4,5,6,7-tetrahydro-2-(2-hydroxyethyl)-5-methyl-4,6-dioxo-2H-pyrazolo[3,4-d]pyrimidin.

Die erfindungsgemäßen Verbindungen der allgemeinen Formel I, I', I" bzw. I‴, worin $R^1$, $R^{1'}$, $R^{1''}$ bzw. $R^{1'''}$ und $R^2$, $R^{2'}$, $R^{2''}$ bzw. $R^{2'''}$ die oben angegebenen Bedeutungen haben und A, A', A" bzw. A‴ die Gruppe

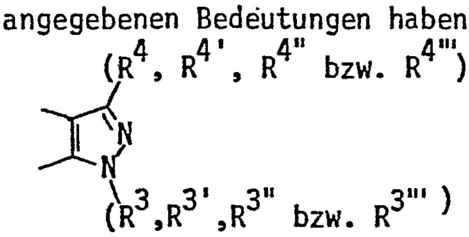

bzw.

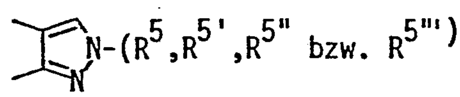

bedeutet, wobei $R^3$, $R^{3'}$, $R^{3''}$ bzw. $R^{3'''}$ und $R^5$, $R^{5'}$, $R^{5''}$ bzw. $R^{5'''}$ die oben angegebenen Bedeutungen haben und $R^4$, $R^{4'}$, $R^{4''}$ bzw. $R^{4'''}$ Wasserstoff bedeutet, werden hergestellt, indem man ein 4-Hydrazinouracil der allgemeinen Formel II bzw. IIa,

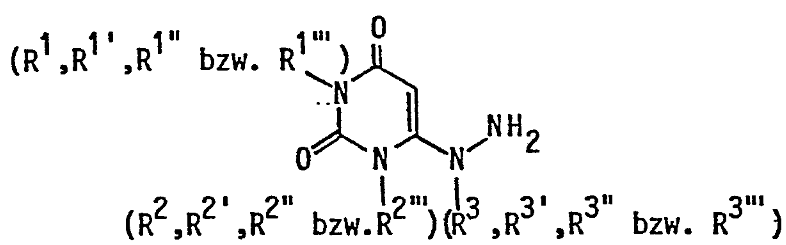

(II),

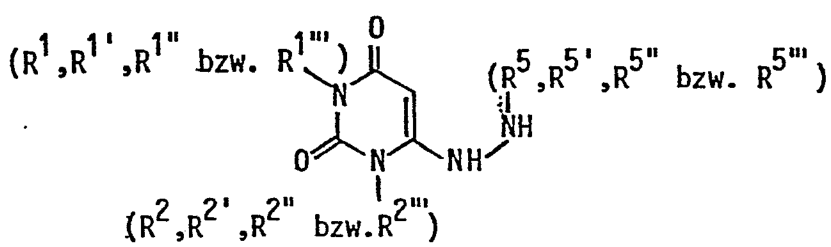

(IIa),

worin $R^1$, $R^{1'}$, $R^{1''}$ bzw. $R^{1'''}$, $R^2$, $R^{2'}$, $R^{2''}$ bzw. $R^{2'''}$, $R^3$, $R^{3'}$, $R^{3''}$ bzw. $R^{3'''}$ und $R^5$, $R^{5'}$, $R^{5''}$ bzw. $R^{5'''}$ die oben angegebenen Bedeutungen haben, mit Phosphoroxychlorid und Dimethylformamid in an sich bekannter Weise, beispielsweise nach dem in der DE-AS 11 86 466 angegebenen Verfahren, umsetzt. Dabei ist es vorteilhaft, das 4-Hydrazinouracil in ein gekühltes Gemisch von Phosphoroxychlorid und Dimethylformamid einzutragen. Nach beendeter Zugabe des 4-Hydrazinouracils läßt man die Reaktionsmischung sich auf Zimmertemperatur erwärmen. Bei Zugabe von Eis fällt das gewünschte Produkt aus.

Das Verfahren zur Herstellung der erfindungsgemäßen Verbindungen der allgemeinen Formeln I, I' bzw. I", worin $R^1$. $R^{1'}$ bzw. $R^{1''}$ und $R^2$, $R^{2'}$ . bzw. $R^{2''}$ die oben angegebenen Bedeutungen haben und A, A' bzw. A" die Gruppe

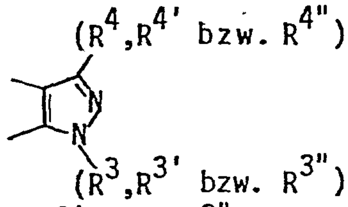

bedeutet, wobei $R^3$, $R^{3'}$ bzw. $R^{3''}$ die oben angegebenen Bedeutungen haben und $R^4$, $R^{4'}$ bzw. $R^{4''}$ die oben angegebenen Bedeutungen mit Ausnahme von Wasserstoff haben, ist dadurch gekennzeichnet, daß ein 4-Hydrazinouracil der allgemeinen Formel II, worin $R^1$, $R^{1'}$ bzw. $R^{1''}$, $R^2$, $R^{2'}$ bzw. $R^{2''}$ und $R^3$, $R^{3'}$. bzw. $R^{3''}$ die oben angegebenen Bedeutungen haben mit einem Aldehyd der Formel ($R^4$,$R^{4'}$ bzw. $R^{4''}$)—$C\genfrac{}{}{0pt}{}{\nearrow O}{\searrow H}$, wobei $R^4$, $R^{4'}$ bzw. $R^{4''}$ die vorstehend angegebenen Bedeutungen haben, umgesetzt wird und das entstandene Hydrazon auf an sich bekannte Weise unter Ringsschluß dehydriert wird. Diese Dehydrierung wird beispielsweise thermolytisch durchgeführt. (F. Yoneda u. T. Nagamatsu, Synthesis, 1973, 300).

Die thermolytische Dehydrierung des Hydrazons wird zweckmäßigerweise bei der Schmelztemperatur des Hydrazons bis 30°C oberhalb der Schmelztemperatur des Hydrazons, vorzugsweise bei 5 bis 20°C oberhalb der Schmelztemperatur des Hydrazons, insbesondere etwa 10°C oberhalb der Schmelztemperatur des Hydrazons durchgeführt. Als in den meisten Fällen günstig hat sich ein Temperaturbereich von 180 bis 220°C erwiesen. Besonders günstig ist eine Temperatur von etwa 200°C. Es ist vorteilhaft, die Thermolyse des Hydrazons in einer Schutzgasatmosphäre, wie beispielsweise unter Stickstoffbegasung durchzuführen. Die Reaktionszeit beträgt im allgemeinen 2 bis 40 Minuten, vorzugsweise 5 bis 20 Minuten. Zur Aufarbeitung wird die Reaktionsmasse aus einem geeigneten Lösungsmittel, wie beispielsweise Ethanol, umkristallisiert.

Erfindungsgemäße Verbindungen mit Alkylthioalkyl-Substituenten werden hergestellt, indem von erfindungsgemäßen Verbindungen ausgegangen wird, die einen Alkenylsubstituenten mit endständiger Doppelbindung aufweisen. An diese Doppelbindung wird das entsprechende Alkylmercaptan auf an sich bekannte Weise angelagert.

Erfindungsgemäße Verbindungen, in denen einer der Reste $R^1$ und $R^2$ einen Alkylcarbonylalkylrest bedeutet, können hergestellt werden, indem ein entsprechendes 4,5,6,7-Tetrahydro-4,6-dioxo-pyrazolo-[3,4-d]pyrimidin mit einem ω,ω'-Dibromalkan alkyliert wird, das erhaltene ω-Bromalkylderivat mit Acetesssigester umgesetzt wird und das Reaktionsprodukt der Ketonspaltung unterworfen wird. Beispielsweise erhält man durch Umsetzung von 7-Ethyl-4,5,6,7-tetrahydro-1-methyl-4,6-dioxo-1H-pyrazolo[3,4-d]pyrimidin mit 1,3-Dibrompropan 5-(3-Brompropyl)-7-ethyl-4,5,6,7-tetrahydro-1-methyl-4,6-dioxo-1H-pyrazolo[3,4-d]pyrimidin, aus dem man durch Umsetzung mit Acetessigester und anschließende Ketonspaltung 7-Ethyl-4,5,6,7-tetrahydro-1-methyl-4,6-dioxo-5-(5-oxo-n-hexyl)-1H-pyrazolo[3,4-d]pyrimidin erhält. Alternativ läßt sich ein Alkylcarbonylalkylrest durch Umsetzung des entsprechenden 4,5,6,7-Tetrahydro-4,6-dioxo-pyrazolo[3,4-d]pyrimidins, vorzugsweise in Form seines Natriumsalzes, mit einem Alkylcarbonylalkylhalogenid, vorzugsweise -chlorid, erhalten. Beispielsweise zur Einführung eines 5-Oxo-n-hexylrests eignet sich 1-Chlorhexanon-5.

Ein weiterer Gegenstand der Erfindung sind die vorstehend genannten Verfahrensvarianten und die in den Patentansprüchen definierten Verfahren zur Herstellung der neuen Verbindungen.

Die Arzneimittel werden nach an sich bekannten Verfahren hergestellt, wobei die neuen Verbindungen als solche oder gegebenenfalls in Kombination mit geeigneten pharmazeutischen Trägerstoffen eingesetzt werden. Enthalten die neuen pharmazeutischen Zubereitungen neben den Wirkstoffen pharmazeutische Trägerstoffe, beträgt der Wirkstoffgehalt dieser Mischungen 0,5 bis 95, vorzugsweise 15 bis 75 Gewichtsprozent der Gesamtmischung.

In Übereinstimmung mit der Erfindung werden die Wirkstoffe in jeder geeigneten Formulierung angewandt unter der Voraussetzung, daß die Ausbildung bzw. Aufrechterhaltung von ausreichenden Wirkstoffspiegeln gewährleistet ist. Das kann beispielsweise durch orale, rektale oder

parenterale Gabe in geeigneten Dosen erreicht werden. Oblicherweise liegt die pharmazeutische Zubereitung des Wirkstoffes in Form von Einheitsdosen vor, die auf die gewünschte Verabreichung abgestimmt sind. Eine Einheitsdosis kann zum Beispiel eine Tablette, ein Dragée, eine Kapsel, ein Suppositorium oder eine gemessene Volumenmenge eines Pulvers, eines Granulates, einer Lösung, einer Emulsion oder einer Suspension sein.

Unter "Einheitsdosis" im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z.B. in Form einer Tablette mit Bruchkerbe.

Die pharmazeutischen Zubereitungen gemäß der Erfindung können, wenn sie in Einheitsdosen vorliegen und für die Applikation z.B. am Menschen bestimmt sind, etwa 10 bis 500 mg, vorteilhafterweise 50 bis 300 mg und insbesondere 100 bis 300 mg Wirkstoff enthalten. Parenterale Zubereitungen können etwa 2 bis 40 mg, vorteilhafterweise 4 bis 30 mg und insbesondere 10 bis 25 mg Wirkstoff enthalten.

Im allgemeinen hat es sich in der Humanmedizin als vorteilhaft erwiesen, den oder die Wirkstoffe bei oraler Gabe in einer Tagesdosis von 0,1 bis 12, vorzugsweise 1 bis 8, insbesondere 2 bis 4 mg/kg Körpergewicht, gegebenenfalls in Form mehrerer, vorzugsweise 1 bis 3 Einzelgaben zur Erzielung der gewünschten Ergebnisse zu verabreichen. Eine Einzelgabe enthält den oder die Wirkstoffe in Mengen von 0,1 bis 6, vorzugsweise 0,5 bis 4, insbesondere 1 bis 3 mg/kg Körpergewicht. Für die bevorzugte inhalative Verabreichung hat es sich als vorteilhaft erwiesen, den oder die Wirkstoffe in einer Tagesdosis von 0,5 bis 10 mg, vorzugsweise 1 bis 8 mg, insbesondere 2 bis 5 mg, gegebenenfalls in Form mehrerer, vorzugsweise 1 bis 3 Einzelgaben zu verabreichen.

Zubereitungen für die intravenöse Verabreichung sind vor allem für die Notfallbehandlung zweckmäßig.

Die therapeutische Verabreichung der pharmazeutischen Zubereitung kann 1 bis 4 mal am Tage zu festgelegten oder variierenden Zeitpunkten erfolgen, z.B. jeweils vor den Mahlzeiten und/oder am Abend. Es kann jedoch erforderlich sein, von den genannten Dosierungen abzuweichen, und zwar in Abhängigkeit von der Art, dem Körpergewicht und dem Alter des zu behandelnden Individuums, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Bei akuten Fällen wird zu Beginn der Behandlung eine höhere Dosis verabreicht. Nach Eintreten der gewünschten Wirkung wird auf eine niedrigere Dosis zurückgegangen.

Die Festlegung der jeweils erforderlichen optimalen Dosierung und Applikationsart der Wirkstoffe kann durch jeden Fachmann aufgrund seines Fachwissens erfolgen.

Die pharmazeutischen Zubereitungen bestehen in der Regel aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z.B. Tabletten, Dragées, harte und weiche Kapseln, z.B. als Gelatine, dispergierbare Pulver, Granulate, wäßrige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Lactose; Granulierungs- und Verteilungsmittel, z.B. Maisstärke oder Alginate; Bindemittel, z.B. Stärke, Gelatine oder Akaziengumme; und Gleitmittel, z.B. Aluminium- oder Magnesiumstearat, Talkum oder Silikonöl, enthalten. Sie können zusätzlich mit einem Überzug versehen sein, der auch so beschaffen sein kann, daß er eine verzögerte Auflösung und Resorption des Arzneimittels im Gastrointestinaltrakt bewirkt, so daß z.B. eine bessere Verträglichkeit, Protrahierung oder eine Retardierung erreicht wird. Gelatinekapseln können den Arzneistoff vermischt mit einem festen, z.B. Calciumcarbonat oder Kaolin, oder einem öligen, z.B. Oliven-, Erdnuß- oder Paraffinöl, Verdünnungsmittel enthalten.

Wäßrige Suspensionen, die gegebenenfalls kurzfristig zubereitet werden, können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycetanol, Polyoxyethylensorbitolmonooleat, Polyoxyethylensorbitanmonooleat oder Lecithin; Konservierungsmittel, z.B. Methyl- oder Propylhydroxybenzoate; Geschmacksmittel; Süßungsmittel, z.B. Saccharose, Lactose, Natriumcyclamat, Dextrose, Invertzuckersirup, enthalten.

Ölige Suspensionen können z.B. Erdnuß-, Oliven-, Sesam-, Kokos- oder Paraffinöl und Verdickungsmittel, wie z.B. Bienenwachs, Hartparaffin oder Cetylalkohol, enthalten; ferner Süßungsmittel, Geschmacksmittel und Antioxidantien.

In Wasser dispergierbare Pulver und Granulate können die Arzneistoffe in Mischung mit Dispergier-, Benetzungs- und Suspendiermitteln, z.B. den oben genannten, sowie mit Süßungsmitteln, Geschmacksmitteln und Farbstoffen enthalten.

Emulsionen können z.B. Oliven-, Erdnuß- oder Paraffinöl neben Emulgiermitteln, wie z.B. Akaziengummi, Traganthgummi, Phosphatiden, Sorbitanmonooleat, Polyoxyethylensorbitanmonooleat, und Süßungs- und Geschmacksmitteln enthalten.

Zur rektalen Anwendung der Arzneistoffe werden Suppositorien verwendet, die mit Hilfe von bei Rektaltemperatur schmelzenden Bindemitteln, beispielsweise Kakaobutter oder Polyethylenglykol, hergestellt werden.

Zur parenteralen Anwendung der Arzneistoffe dienen steril injizierbare, gegebenenfalls kurzfristig herzustellende, wäßrige Suspensionen, isotonische Salzlösungen oder sonstige Lösungen, die Dispergier- oder Benetzungsmittel und/oder pharmakologisch verträgliche Verdünnungsmittel, z.B. Propylen- oder Butylenglykol, enthalten.

Die orale Anwendung der Arzneistoffe ist bevorzugt.

Bevorzugt ist auch die inhalative Applikation der erfindungsgemäßen Verbindungen. Diese werden entweder direkt als Pulver oder durch Vernebeln von die erfindungsgemäßen Verbindungen enthaltenden Lösungen oder Suspensionen verabreicht. Die Verneblung kann dabei auf herkömmliche Weise, beispielsweise durch Preßluftvernebler oder Ultraschallvernebler, erfolgen. Besonders vorteilhaft ist die Verabreichung aus Sprühdosen, insbesondere solcher mit einem herkömmlichen Dosierventil (Dosier-Aerosole). Mittels Dosier-Aerosolen ist es möglich, pro Sprühstoß eine definierte Menge an Wirkstoff bereitzustellen. Von besonderem Vorteil sind hier sogenannte Synchron-Inhalatoren, womit die Wirkstoffabgabe synchron zur Einatmung geschehen kann. Geeignete Synchron-Inhalationsvorrichtungen sind z.B. in der DE-PS 19 45 257, DE-PS 19 17 911 und DE-OS 20 55 734 offenbart.

Für Inhalationszwecke kommen die Wirkstoffe vorzugsweise in mikronisierter Form zum Einsatz, wobei Teilchengrößen von weniger als 10μm vorteilhaft sind. Zur Applikation aus Sprühdosen werden die Wirkstoffe in üblichen Treibmitteln dispergiert, vorzugsweise unter Zuhilfenahme eines Dispergiermittels. Als Treibmittel kommen insbesondere Gemische von Trichlorfluormethan (Frigen®11) und Dichlordifluormethan (Frigen®12) in Frage, wobei Trichlorfluormethan ganz oder teilweise durch 1,1,2-Trichlortrifluormethan (Frigen®113) ersetzt werden kann. Als Dispergiermittel kommen insbesondere die für diese Zwecke gebräuchlichen Sorbitanester (Spane® der Firma Atlas GmbH) und Lecithin in Frage. Das Dispergiermittel wird in der

gekühlt vorgelegten schwerer flüchtigen Treibmittelkomponente gelöst. In die Lösung wird der mikronisierte Wirkstoff bzw. werden die mikronisierten Wirkstoffe eingerührt. Die Dispersion wird in Sprühbüchsen eingefüllt. Nach dem Vercrimpen wird die leichter flüchtige Treibmittelkomponente aufgedrückt.

Der oder die Wirkstoffe können gegebenenfalls mit einem oder mehreren der angegebenen Träger- oder Zusatzstoffe auch in mikroverkapselter Form formuliert werden.

Die erfindungsgemäßen Verbindungen zeigen u.a. eine starke Phosphodiesterasehemmung und antagonisieren die durch Methanol hervorgerufene Schädigung der Netzhaut.

In der nachfolgenden Tabelle wird den untersuchten Verbindungen eine laufende römische Nummer zugeordnet.

Tabelle:

| | 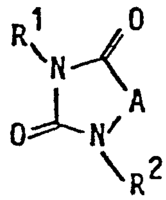 , wobei A 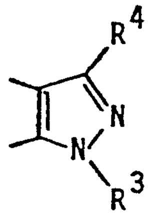 oder 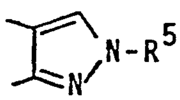 | | | | |
|---|---|---|---|---|---|
| Verbindung Nr. | $R^1$ | $R^2$ | $R^3$ | $R^4$ | $R^5$ |
| I | $CH_3$ | $n-C_6H_{13}$ | H | H | - |
| II | $CH_3$ | $n-C_6H_{13}$ | - | - | $CH_3$ |
| III | $CH_3$ | $n-C_6H_{13}$ | - | - | $C_2H_5$ |
| IV | $CH_3$ | $n-C_6H_{13}$ | - | - | $n-C_3H_7$ |
| V | $CH_3$ | $n-C_6H_{13}$ | - | - | $n-C_4H_9$ |
| VI | $CH_3$ | $n-C_6H_{13}$ | - | - | $n-C_6H_{13}$ |
| VII | $n-C_6H_{13}$ | $CH_3$ | H | H | - |
| VIII | $n-C_6H_{13}$ | $CH_3$ | - | - | $CH_3$ |
| IX | $n-C_6H_{13}$ | $CH_3$ | - | - | $C_2H_5$ |
| X | $n-C_6H_{13}$ | $CH_3$ | - | - | $n-C_4H_9$ |
| XI | $n-C_6H_{13}$ | $CH_3$ | - | - | $n-C_6H_{13}$ |
| XII | $n-C_6H_{13}$ | $CH_3$ | - | - | 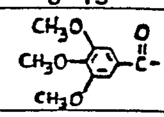 |
| XIII | $CH_3$ | $n-C_6H_{13}$ | $CH_3$ | H | - |
| XIV | $n-C_6H_{13}$ | $CH_3$ | $CH_3$ | H | - |
| XV | $CH_3$ | $CH_2-CH{<}^{CH_3}_{CH_3}$ | H | H | - |
| XVI | $CH_2CH{<}^{CH_3}_{CH_3}$ | $CH_3$ | - | | 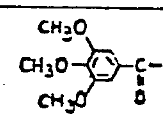 |
| XVII | $CH_3\underset{\overset{\|}{O}}{C}-(CH_2)_4-$ | $C_2H_5$ | $CH_3$ | H | - |
| XVIII | $CH_3$ | $i-C_4H_9$ | - | - | $HOCH_2CH_2$ |

Die Tabelle 1 gibt die gefundenen Werte der Hemmung der Phosphodiesterase (PDE) an. Es wurde Rinderherz PDE "Boehringer" in Glycerin und als Substrat cAMP (0,15 mM) verwendet. Die Konzentration der untersuchten Verbindungen war 0,2 mM.

Tabelle 1

| Verbindung Nr. | Hemmung der PDE in % |
|---|---|
| I | 47,84 |
| II | 47,41 |
| III | 58,51 |
| IV | 63,04 |
| V | 70,86 |
| VI | 37,30 |
| VII | 52,43 |
| VIII | 42,04 |
| IX | 71,52 |
| X | 37,83 |
| XI | 18,35 |
| Theophyllin | 37,13 |

In Tabelle 2 ist die retinotrope Wirkung, gemessen am Elektroretinogramm der Maus, für erfindungsgemäße Verbindungen wiedergegeben. Die Versuchsmethodik ist bei K.K.Gauri und W.Hohl, Der Augenspiegel, 1979 (Heft 9), und den darin angegebenen Referenzen beschrieben.

Tabelle 2:

| Verbindung Nr. | Dosis [mg/kg] | Retinotrope Wirkung [%] |
|---|---|---|
| III | 1 | +24 |
| V | 10 | + 6 |
| IX | 1 | +12 |
| XII | 1 | +40 |
| XVII | 2 | +80 |
| Theophyllin | 10 | 0 |
| Pentoxifyllin | 6,25 | 0 |

Die erfindungsgemäßen Verbindungen antagonisieren die durch Methanol hervorgerufene Retinaschädigung (retinotrope Wirkung), während bei Theophyllin und Pentoxifyllin zumindest in dem Dosisbereich, in dem die erfindungsgemäßen Verbindungen untersucht wurden, noch keine retinotrope Wirkung zeigen.

Weiterhin wurde die relaxierende Wirkung erfindungsgemäßer Verbindungen auf die Trachealspangen-Kette des Meerschweinchens in vitro geprüft:

Vier parallele, aus jeweils 6 Einzelringen bestehende Trachealspangen-Ketten des Meerschweinchens (♂ und ♀, 430-600 g) im Organbad (5 ml, Krebs-Henseleit-Lösung mit Zusatz von Phentolamin ($10^{-5}$ mol/l, 37°C, Vorspannung der Organe 2 g, Begasung mit Carbogen) entwickeln nach etwa 20 bis 30 Minuten eine stabile, tonische Spontankontraktur. An diesen dauerkontrahierten Organen kann unter isometrischen Meßbedingungen durch Applikation der Prüfsubstanz in kumulativhalblogarithmisch ansteigender Konzentration (z.B. $1x10^{-6}$ + $2x10^{-6}$ + $7x10^{-6}$ + $2x10^{-5}$ usw. mol/l) eine Relaxation herbeigeführt werden, wobei nach jeder Einzeldosis der Testsubstanz eine konstante Relaxations-Antwort abgewartet wird, bevor die nächst höhere Konzentration appliziert wird. Über einen Zeitraum von 20 bis 30 Minuten wird somit eine vollständige Dosis-Wirkungskurve der Testsubstanz erhalten. Die jeweilige Relaxation wird als Prozentbruchteil der durch Gabe von (-)Isoprenalin ($10^{-6}$ mol/l) maximal erreichbaren Relaxation ausgedrückt.

In Tabelle 3 sind die $ED_{50}$ (Dosis der halbmaximal möglichen Relaxation) und die relative Wirkungsstärke im Vergleich zu Theophyllin für erfindungsgemäße Verbindungen angegeben.

Tabelle 3:

| Verbindung Nr. | $ED_{50}$[mol/l] | Relative Wirkungsstärke (Theophyllin=1,00) |
|---|---|---|
| V | $6{,}2 \times 10^{-5}$ | 1,8 |
| IX | $3{,}8 \times 10^{-5}$ | 3,4 |
| XII | $3{,}5 \times 10^{-4}$ | 3,7 |
| XIII | $9{,}5 \times 10^{-5}$ | 1,4 |
| XV | $1{,}7 \times 10^{-5}$ | 7,6 |
| XVI | $7{,}5 \times 10^{-5}$ | 1,7 |
| XVII | $6{,}6 \times 10^{-5}$ | 2,0 |
| XVIII | $1{,}3 \times 10^{-5}$ | 10,0 |
| Theophyllin | $1{,}3 \times 10^{-4}$ | 1,00 |

Beispiele

1. 5-n-Hexyl-4,5,6,7-tetrahydro-7-methyl-4,6-dioxo-1H-pyrazolo[3,4-d]-pyrimidin

In ein Gemisch aus 3 g Phosphoroxychlorid und 10 ml Dimethylformamid werden 3 g 1-Hexyl-3-methyl-4-hydrazinouracil unter äußerer Eiskühlung eingetragen. Nach dem Abklingen der exothermen Reaktion läßt man das Reaktionsgemisch auf Zimmertemperatur kommen und rührt bei dieser Temperatur noch eine halbe bis eine Stunde lang weiter. Danach wird genügend Eis zugegeben, um das überschüssige Phosphoroxychlorid zu zersetzen, wobei das gewünschte Produkt in kristalliner Form ausfällt. Analog erhält man die in 1-Stellung substituierten Verbindungen, indem man von Hydrazinouracilen ausgeht, die am α-N-Atom der Hydrazinogruppe den entsprechenden $R^3$-Rest haben.

2. 2-Ethyl-5-n-hexyl-4,5,6,7-tetrahydro-7-methyl-4,6-dioxo-2H-pyrazolo-[3,4-d]pyrimidin

2,5 g des Produktes des Beispiels 1 werden in ca. 20 ml Aceton suspendiert und mit einem Überschuß an Ethylbromid versetzt. Zur Bindung der freiwerdenden Halogenwasserstoffsäure wird Kaliumcarbonat zugegeben, das Ganze wird bis zur Vervollständigung der Umsetzung einige Stunden am Rückfluß gekocht. Anschließend werden das überschüssige Alkylierungsmittel und das Lösungsmittel abdestilliert und der Rückstand in Ethanol-Wasser-Gemisch umkristallisiert. Die erhaltene Titelverbindung schmilzt bei 107 bis 108°C.

Analog können die anderen in 2-Stellung substituierten Verbindungen hergestellt werden.

3. 4,5,6,7-Tetrahydro-7-(2-hydroxyethylthiopropyl)-5-methyl-4,6-dioxo-1H-pyrazolo[3,4-d]pyrimidin

2,50 g 1-Allyl-6-chlor-3-methyluracil werden in überschüssigem Mercaptoethanol aufgelöst. Sobald sich dünnschichtchromatographisch ($CHCl_3$/Petrolether 1:1) kein Ausgangsprodukt ($R_f \sim 0,2$) mehr nachweisen läßt, wird die Lösung unter vermindertem Druck eingeengt. Der Rückstand wird in 30 %iger wäßriger Hydrazinlösung gelöst. 2 g des nach einiger Zeit ausfallenden Produktes (Schmelzpunkt 227 bis 228°C) werden unter Kühlung in 13 ml Dimethylformamid und 2 ml $POCl_3$ gelöst. Die Lösung wird 30 Minuten

bei Zimmertemperatur gerührt. Der auf Zugabe von Wasser ausfallende Niederschlag wird abfiltriert und aus Methanol/Wasser umkristallisiert (Schmelzpunkt 273°C).

Analog erhält man die in 2-Stellung substituierten Verbindungen dieses Typs.

4. <u>7-Hexyl-4,5,6,7-tetrahydro-1,5-dimethyl-4,6-dioxo-1H-pyrazolo-[3,4-d]pyrimidin</u>

9 g 3-Hexyl-1-methyl-4-α-N-methylhydrazinouracil werden in ein Gemisch von 9 g Phosphoroxychlorid und 32 ml Dimethylformamid langsam eingetragen, wobei die Reaktionstemperatur durch äußere Kühlung etwa bei 50°C gehalten wird. Nach 20 Minuten Stehen erstarrt das Gemisch zu einem Kristallbrei. Man versetzt mit Eiswasser, filtriert die ausgeschiedenen Kristalle ab und wäscht mit wenig Wasser nach. Nach einmaligem Umkristallisieren erhält man 9,5 g chromatographisch reine Substanz mit einem Schmelzpunkt von 106-107°C.

5. <u>4,5,6,7-Tetrahydro-1,5-dimethyl-4,6-dioxo-7-(5-oxo-n-hexyl)-1H-pyrazolo[3,4-d]pyrimidin</u>

2,7 g 4,5,6,7-Tetrahydro-1,5-dimethyl-4,6-dioxo-1H-pyrazolo[3,4-d]pyrimidin werden zusammen mit 0,6 g Natriumhydroxid in einem Gemisch von 20 ml Ethanol und 10 ml Wasser gelöst. Zu dieser Lösung gibt man 4 ml 1-Chlorhexan-5-on gelöst in Ethanol. Das Reaktionsgemisch wird 3 Stunden am Rückfluß erhitzt.

6. <u>5-n-Hexyl-4,5,6,7-tetrahydro-2-(3,4,5-trimethoxybenzoyl)-7-methyl-4,6-dioxo-2H-pyrazolo[3,4-d]pyrimidin</u>

1-Hexyl-4-hydrazino-3-methyluracil wird in Chloroform/Pyridin 12 Stunden bei 60°C mit 3,4,5-Trimethoxybenzoylchlorid zur Reaktion gebracht. 2,5 g des Reaktionsproduktes werden mit 10 ml Dimethylformamid und 4 g $POCl_3$ umgesetzt. Zur erhaltenen Lösung gibt man Eis und neutralisiert anschliessend mit Natriumcarbonat. Die erhaltene Lösung wird mit Chloroform extrahiert. Die organische Phase wird eingeengt. Der Rückstand wird aus einem Methanol/Wasser-Gemisch umkristallisiert.

7. 5-n-Hexyl-4,5,6,7-tetrahydro-3-(3,4,5-trimethoxyphenyl)-1,7-dimethyl-4,6-dioxo-1H-pyrazolo[3,4-d]pyrimidin

1-n-Hexyl-3-methyl-4-(1-methylhydrazino)-uracil wird bei Zimmertemperatur unter Rühren in Ethanol mit 3,4,5-Trimethoxybenzaldehyd umgesetzt. Das ausgefallene Produkt wird abfiltriert und etwa eine halbe Stunde auf 200°C erhitzt. Nach dem Abkühlen wird aus Ethanol umkristallisiert.

Analog werden die in 3-Stellung durch Ethyl bzw. Phenyl substituierten Verbindungen durch Einsatz von Propionaldehyd bzw. Benzaldehyd dargestellt.

8. 7-Ethyl-4,5,6,7-tetrahydro-1-methyl-4,6-dioxo-5-(5-oxo-n-hexyl)-1H-pyrazolo[3,4-d]pyrimidin

23 g (0,1 Mol) des Natriumsalzes von 7-Ethyl-4,5,6,7-tetrahydro-1-methyl-4,6-dioxo-1H-pyrazolo[3,4-d]pyrimidin werden in 150 ml Wasser unter Erwärmen auf 50°C gelöst. Bei Siedehitze tropft man sodann langsam 1-Chlorhexanon-5 zu und erhitzt anschließend 3 Stunden am Rückfluß. Das Reaktionsgemisch wird dann unter Vakuum zur Trockne eingedampft. Der Rückstand wird mit 250 ml Chloroform aufgenommen und zweimal mit jeweils 50 ml 2 %iger Natronlauge extrahiert. Die organische Phase wird mit Wasser nachgewaschen und anschließend über Natriumsulfat getrocknet. Nach Abziehen des Lösungsmittels erhält man 19 g der Titelverbindung (65 % der Theorie). Nicht umgesetztes Ausgangsprodukt kann aus der alkalischen wäßrigen Phase zurückgewonnen werden (Schmelzpunkt 111,5°C).

9. 7-i-Butyl-4,5,6,7-tetrahydro-2-(2-hydroxyethyl)-5-methyl-4,6-dioxo-2H-pyrazolo[3,4-d]pyrimidin

5,4 g 7-i-Butyl-4,5,6,7-tetrahydro-5-methyl-4,6-dioxo-pyrazolo[3,4-d]-pyrimidin werden mit überschüssigem Chlorethanol zusammen mit 9 g Kaliumcarbonat in Dimethylformamid viereinhalb Stunden am Rückfluß erhitzt. Das Reaktionsgemisch wird filtriert und das Lösungsmittel abdestilliert. Der Rückstand wird aus Wasser umkristallisiert (Ausbeute 4 g, Schmelzpunkt 157,5 bis 158,5°C).

Analog wird 7-Ethyl-4,5,6,7-tetrahydro-2-(2-hydroxyethyl)-5-methyl-4,6-dioxo-2H-pyrazolo[3,4-d]pyrimidin aus 7-Ethyl-4,5,6,7-tetrahydro-5-methyl-4,6-dioxo-pyrazolo[3,4-d]pyrimidin hergestellt.

10. Tabletten mit 100 mg 7-i-Butyl-4,5,6,7-tetrahydro-5-methyl-4,6-dioxo-1H-pyrazolo[3,4-d]pyrimidin

40,000 kg Wirkstoff, 24,000 kg Milchzucker und 16,000 kg Maisstärke werden mit 4,000 kg Polyvinylpyrrolidon (MG ~25.000) in ungefähr 5,5 Liter Wasser granuliert und durch ein Sieb von 1,25 mm Maschenweite gepreßt. Nach dem Trocknen werden 10,000 kg Carboxymethylcellulose, 4,000 kg Talkum und 2,000 kg Magnesiumstearat zugegeben. Auf einer Exzentermaschine wird das Granulat zu Tabletten von 9 mm Durchmesser, 250 mg Gewicht und einer Härte von 4 bis 5 kg verpreßt.

11. Dosieraerosolzubereitung enthaltend 5-n-Hexyl-4,5,6,7-tetrahydro-2-(3,4,5-trimethoxybenzoyl)-7-methyl-4,6-dioxo-2H-pyrazolo-[3,4-d]pyrimidin

0,540 g Span® 85 und 0,135 g Aroma werden in 10,215 g gekühltem Frigen® 11 gelöst. In die Lösung werden 0,270 g mikronisierter Wirkstoff eingerührt und in 24 ml-Dosen eingefüllt. Nach dem Vercrimpen werden 14,971 g Frigen® 12 eingepreßt. Bei einem Kammervolumen des Dosierventils von 125 µl werden pro Ventilhub 1,6 mg Wirkstoff als Aerosol freigesetzt.

Patentansprüche

1. Pyrazolo[3,4-d]pyrimidine der allgemeinen Formel I

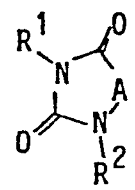

(I),

worin

$R^1$ und $R^2$, die gleich oder verschieden sind, für Wasserstoff oder die Reste $C_1$-$C_8$-Alkyl, $C_2$-$C_8$-Alkenyl, $C_4$-$C_8$-Alkylcarbonylalkyl, $C_3$-$C_8$-Alkylthioalkyl und $C_3$-$C_8$-Alkoxyalkyl, die durch Halogen, Hydroxy oder den Di($C_1$-$C_2$-Alkyl)aminorest substituiert sein können, $R^1$ und $R^2$ jedoch nicht gleichzeitig Wasserstoff sein können, und

A für eine Gruppe

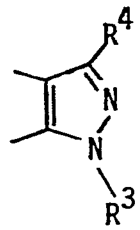

oder

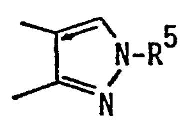

,

wobei

$R^3$ Wasserstoff oder $C_1$-$C_4$-Alkyl,

$R^4$ Wasserstoff, $C_1$-$C_4$-Alkyl oder Phenyl, das bis zu dreifach $C_1$-$C_2$-alkoxysubstituiert sein kann,

$R^5$ Wasserstoff, $C_1$-$C_6$-Alkyl, Hydroxy-$C_1$-$C_3$-alkyl, Di($C_1$-$C_2$-alkyl)amino-$C_1$-$C_4$-alkyl, $C_2$-$C_6$-Alkenyl, Phenyl, Phenyl-$C_1$-$C_2$-Alkyl oder Benzoyl, wobei die Phenylringe der letztgenannten drei Reste bis zu dreifach $C_1$-$C_2$-alkoxysubstituiert sein können, $C_1$-$C_3$-Alkylthio-$C_2$-$C_4$-alkyl oder Hydroxy-, Amino- oder Sulfo-$C_1$-$C_3$-alkylthio-$C_2$-$C_4$-alkyl bedeuten,

stehen, wobei $R^1$, $R^2$ und $R^5$ nicht gleichzeitig Ethyl bedeuten, $R^1$ und $R^2$ nicht beide Methyl bedeuten, wenn $R^3$ oder $R^5$ Butyl oder Di($C_1$-$C_2$-alkyl)aminoethyl bedeuten, und die Summe der Kohlenstoff-, Sauerstoff-, Stickstoff- und Schwefelatome der an Ringstickstoffatome gebundenen

Ketten zusammen mindestens 6 beträgt, oder mindestens 5 beträgt, wenn $R^2$ Isobutyl bedeutet.

2. Pyrazolo[3,4-d]pyrimidine der allgemeinen Formel I',

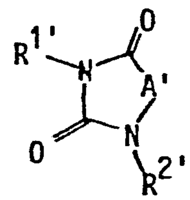

(I'),

worin

$R^{1'}$ und $R^{2'}$, die gleich oder verschieden sind, für Wasserstoff oder die Reste $C_1$-$C_8$-Alkyl, $C_2$-$C_4$-Alkenyl, $C_5$-$C_7$-Alkylcarbonylalkyl, $C_5$-$C_7$-Alkylthioalkyl und $C_3$-$C_7$-Alkoxyalkyl, die durch Hydroxy oder den Dimethylaminorest substituiert sein können, $R^{1'}$ und $R^{2'}$ jedoch nicht gleichzeitig Wasserstoff sein können, und

A' für eine Gruppe

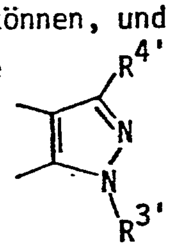

oder

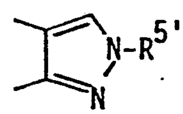

,

wobei

$R^{3'}$ Wasserstoff oder $C_1$-$C_3$-Alkyl

$R^{4'}$ Wasserstoff, $C_1$-$C_3$-Alkyl oder Phenyl, das bis zu dreifach methoxysubstituiert sein kann, und

$R^{5'}$ Wasserstoff, $C_1$-$C_6$-Alkyl, Hydroxy-$C_1$-$C_3$-alkyl, Di($C_1$-$C_2$-alkyl)aminoethyl, $C_2$-$C_3$-Alkenyl, Phenyl, Benzyl, Benzoyl, wobei die Phenylringe der letztgenannten drei Reste durch bis zu drei Methoxygruppen substituiert sein können, oder Hydroxy-$C_1$-$C_2$-alkylthio-$C_2$-$C_3$-alkyl bedeuten,

stehen, wobei $R^{1'}$, $R^{2'}$ und $R^{5'}$ nicht gleichzeitig Ethyl bedeuten, $R^{1'}$ und $R^{2'}$ nicht beide Methyl bedeuten, wenn $R^{5'}$ Butyl oder Di($C_1$-$C_2$-alkyl)-aminoethyl bedeutet, und die Summe der Kohlenstoff-, Sauerstoff-, Stickstoff- und Schwefelatome der an Ringstickstoffatome gebundenen Ketten zusammen mindestens 6 beträgt, oder mindestens 5 beträgt, wenn $R^{2'}$ Isobutyl bedeutet.

3. Pyrazolo[3,4-d]pyrimidine der allgemeinen Formel I",

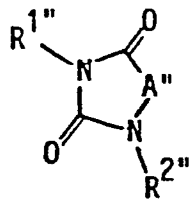

(I"),

worin

$R^{1''}$ und $R^{2''}$, die gleich oder verschieden sind, für Wasserstoff, $C_1$-$C_6$-Alkyl, 2-Hydroxyethyl, Allyl, 5-Oxo-n-hexyl, Hydroxyethoxymethyl, Hydroxyethoxy-n-propyl, Hydroxyethylthio-n-propyl oder 2-(Dimethylamino)ethyl, $R^{1''}$ und $R^{2''}$ jedoch nicht gleichzeitig Wasserstoff sein können,

und A" für eine Gruppe

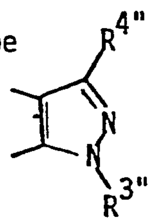

oder

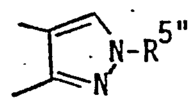

wobei

$R^{3''}$ Wasserstoff, Methyl oder Ethyl,

$R^{4''}$ Wasserstoff oder Phenyl und

$R^{5''}$ Wasserstoff, $C_1$-$C_6$-Alkyl, 2-Hydroxyethyl, Allyl, Trimethoxyphenyl, Trimethoxybenzoyl oder Hydroxyethylthio-n-propyl bedeuten,

stehen, wobei $R^{1''}$, $R^{2''}$ und $R^{5''}$ nicht gleichzeitig Ethyl bedeuten, $R^{1''}$ und $R^{2''}$ nicht beide Methyl bedeuten, wenn $R^{5''}$ Butyl bedeutet, und die Summe der Kohlenstoff-, Sauerstoff-, Stickstoff- und Schwefelatome der an Ringstickstoffatome gebundenen Ketten zusammen mindestens 6 beträgt, oder mindestens 5 beträgt, wenn $R^{2''}$ Isobutyl bedeutet.

4. Pyrazolo[3,4-d]pyrimidine der allgemeinen Formel I''',

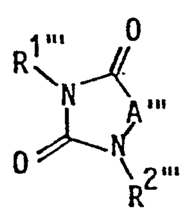

(I'''),

worin einer der Reste $R^{1'''}$ und $R^{2'''}$ für Wasserstoff, Methyl oder Ethyl, der andere für Ethyl, Butyl, Hexyl, 5-Oxo-n-hexyl oder 2-(Dimethylamino)-ethyl, und

A''' für eine Gruppe 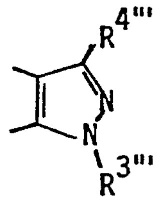 oder 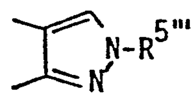

wobei

$R^{3'''}$ Wasserstoff oder Methyl,

$R^{4'''}$ Wasserstoff und

$R^{5'''}$ $C_1$-$C_6$-Alkyl, 2-Hydroxyethyl und Trimethoxybenzoyl bedeuten,

stehen, wobei die Summe der Kohlenstoff-, Sauerstoff- und Stickstoffatome der an Ringstickstoffatome gebundenen Ketten zusammen mindestens 6 beträgt, oder mindestens 5 beträgt, wenn $R^{2'''}$ Isobutyl bedeutet.

5. 7-i-Butyl-4,5,6,7-tetrahydro-5-methyl-4,6-dioxo-1H-pyrazolo-[3,4-d]pyrimidin.

6. 2-n-Butyl-7-n-hexyl-4,5,6,7-tetrahydro-5-methyl-4,6-dioxo-2H-pyrazolo[3,4-d]pyrimidin.

7. 2-Ethyl-5-n-hexyl-4,5,6,7-tetrahydro-7-methyl-4,6-dioxo-2H-pyrazolo[3,4-d]pyrimidin.

8. 5-n-Hexyl-4,5,6,7-tetrahydro-2-(3,4,6-trimethoxybenzoyl)-7-methyl-4,6-dioxo-2H-pyrazolo[3,4-d]pyrimidin.

9. 7-Ethyl-4,5,6,7-tetrahydro-1-methyl-4,6-dioxo-5-(5-oxo-n-hexyl)-1H-pyrazolo[3,4-d]pyrimidin.

10. 7-i-Butyl-4,5,6,7-tetrahydro-2-(2-hydroxyethyl)-5-methyl-4,6-dioxo-2H-pyrazolo[3,4-d]pyrimidin.

11. 7-Ethyl-4,5,6,7-tetrahydro-2-(2-hydroxyethyl)-5-methyl-4,6-dioxo-2H-pyrazolo[3,4-d]pyrimidin.

12. Verfahren zur Herstellung der Pyrazolo[3,4-d]pyrimidine der allgemeinen Formel I, 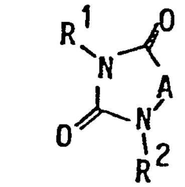 (I),

worin

$R^1$ und $R^2$, die gleich oder verschieden sind, für Wasserstoff oder die Reste $C_1$-$C_8$-Alkyl, $C_2$-$C_8$-Alkenyl, $C_4$-$C_8$-Alkylcarbonylalkyl, $C_3$-$C_8$-Alkylthioalkyl und $C_3$-$C_8$-Alkoxyalkyl, die durch Halogen, Hydroxy oder den Di($C_1$-$C_2$-Alkyl)aminorest substituiert sein können, $R^1$ und $R^2$ jedoch nicht gleichzeitig Wasserstoff sein können, und

A für eine Gruppe 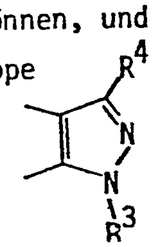 oder 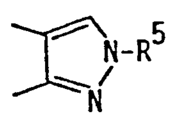,

wobei

$R^3$ Wasserstoff oder $C_1$-$C_4$-Alkyl,

$R^4$ Wasserstoff, $C_1$-$C_4$-Alkyl oder Phenyl, das bis zu dreifach $C_1$-$C_2$-alkoxysubstituiert sein kann,

$R^5$ Wasserstoff, $C_1$-$C_6$-Alkyl, Hydroxy-$C_1$-$C_3$-alkyl, Di($C_1$-$C_2$-alkyl)amino-$C_1$-$C_4$-alkyl, $C_2$-$C_6$-Alkenyl, Phenyl, Phenyl-$C_1$-$C_2$-Alkyl oder Benzoyl, wobei die Phenylringe der letztgenannten drei Reste bis zu dreifach $C_1$-$C_2$-alkoxysubstituiert sein können, $C_1$-$C_3$-Alkylthio-$C_2$-$C_4$-alkyl oder Hydroxy-, Amino- oder Sulfo-$C_1$-$C_3$-alkylthio-$C_2$-$C_4$-alkyl bedeuten,

stehen, wobei $R^1$, $R^2$ und $R^5$ nicht gleichzeitig Ethyl bedeuten, $R^1$ und $R^2$ nicht beide Methyl bedeuten, wenn $R^3$ oder $R^5$ Butyl oder Di($C_1$-$C_2$-alkyl)aminoethyl bedeuten, und die Summe der Kohlenstoff-, Sauerstoff-, Stickstoff- und Schwefelatome der an Ringstickstoffatome gebundenen Ketten zusammen mindestens 6 beträgt, oder mindestens 5 beträgt, wenn $R^2$ Isobutyl bedeutet, dadurch gekennzeichnet, daß man ein 4-Hydrazinouracil der allgemeinen Formel III

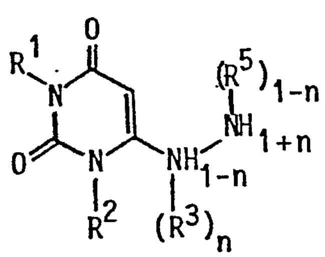 (III),

worin $R^1$, $R^2$, $R^3$ und $R^5$ die vorstehenden Bedeutungen haben und n die Zahl 1 oder 0 bedeutet, mit einem Aldehyd $R^4-C\genfrac{}{}{0pt}{}{O}{H}$, wobei $R^4$ die vorstehenden Bedeutungen hat, umsetzt und das entstandene Hydrazon dehydrierend cyclisiert bzw., wenn $R^4$ in Formel I die Bedeutung Wasserstoff hat, das 4-Hydrazinouracil der allgemeinen Formel III mit Phosphoroxychlorid und Dimethylformamid umsetzt.

13. Verfahren zur Herstellung der Pyrazolo[3,4-d]pyrimidine der allgemeinen Formel I'

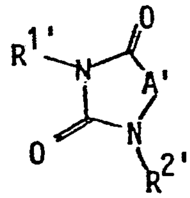

(I'),

worin

$R^{1'}$ und $R^{2'}$, die gleich oder verschieden sind, für Wasserstoff oder die Reste $C_1$-$C_8$-Alkyl, $C_2$-$C_4$-Alkenyl, $C_5$-$C_7$-Alkylcarbonylalkyl, $C_5$-$C_7$-Alkylthioalkyl und $C_3$-$C_7$-Alkoxyalkyl, die durch Hydroxy oder den Dimethylaminorest substituiert sein können, $R^{1'}$ und $R^{2'}$ jedoch nicht gleichzeitig Wasserstoff sein können, und

A' für eine Gruppe

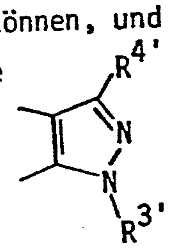

oder

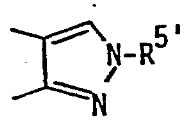

,

wobei

$R^{3'}$ Wasserstoff oder $C_1$-$C_3$-Alkyl

$R^{4'}$ Wasserstoff, $C_1$-$C_3$-Alkyl oder Phenyl, das bis zu dreifach methoxysubstituiert sein kann, und

$R^{5'}$ Wasserstoff, $C_1$-$C_6$-Alkyl, Hydroxy-$C_1$-$C_3$-alkyl, Di($C_1$-$C_2$-alkyl)aminoethyl, $C_2$-$C_3$-Alkenyl, Phenyl, Benzyl, Benzoyl, wobei die Phenylringe der letztgenannten drei Reste durch bis zu drei Methoxygruppen substituiert sein können, oder Hydroxy-$C_1$-$C_2$-alkyl-

thio-$C_2$-$C_3$-alkyl bedeuten,

stehen, wobei $R^{1'}$, $R^{2'}$ und $R^{5'}$ nicht gleichzeitig Ethyl bedeuten, $R^{1'}$ und $R^{2'}$ nicht beide Methyl bedeuten, wenn $R^{5'}$ Butyl oder Di($C_1$-$C_2$-alkyl)aminoethyl bedeutet, und die Summe der Kohlenstoff-, Sauerstoff-, Stickstoff- und Schwefelatome der an Ringstickstoffatome gebundenen Ketten zusammen mindestens 6 beträgt, oder mindestens 5 beträgt, wenn $R^{2'}$ Isobutyl bedeutet, dadurch gekennzeichnet, daß man ein 4-Hydrazinouracil der allgemeinen Formel III',

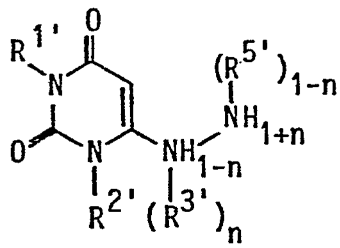

(III'),

worin $R^{1'}$, $R^{2'}$, $R^{3'}$ und $R^{5'}$ die vorstehenden Bedeutungen haben und n die Zahl 1 oder 0 bedeutet, mit einem Aldehyd $R^{4'}-C^{\nearrow O}_{\searrow H}$, wobei $R^{4'}$ die vorstehenden Bedeutungen hat, umsetzt und das entstandene Hydrazon dehydrierend cyclisiert bzw., wenn $R^{4'}$ in Formel I' die Bedeutung Wasserstoff hat, das 4-Hydrazinouracil der allgemeinen Formel III' mit Phosphoroxychlorid und Dimethylformamid umsetzt.

14. Verfahren zur Herstellung der Pyrazolo[3,4-d]pyrimidine der allgemeinen Formel I"

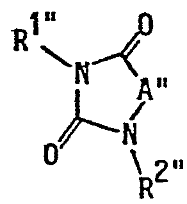

(I"),

worin

$R^{1"}$ und $R^{2"}$, die gleich oder verschieden sind, für Wasserstoff, $C_1$-$C_6$-Alkyl, 2-Hydroxyethyl, Allyl, 5-Oxo-n-hexyl, Hydroxyethoxymethyl, Hydroxyethoxy-n-propyl, Hydroxyethylthio-n-propyl oder 2-(Dimethylamino)ethyl, $R^{1"}$ und $R^{2"}$ jedoch nicht gleichzeitig Wasserstoff sein können,

und A" für eine Gruppe

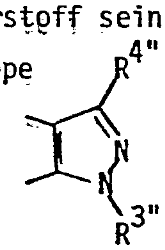

oder

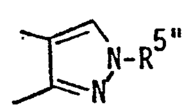

wobei

$R^{3''}$ Wasserstoff, Methyl oder Ethyl,

$R^{4''}$ Wasserstoff oder Phenyl und

$R^{5''}$ Wasserstoff, $C_1$-$C_6$-Alkyl, 2-Hydroxyethyl, Allyl, Trimethoxyphenyl, Trimethoxybenzoyl oder Hydroxyethylthio-n-propyl bedeuten,

stehen, wobei $R^{1''}$, $R^{2''}$ und $R^{5''}$ nicht gleichzeitig Ethyl bedeuten, $R^{1''}$ und $R^{2''}$ nicht beide Methyl bedeuten, wenn $R^{5''}$ Butyl bedeutet, und die Summe der Kohlenstoff, Sauerstoff-, Stickstoff- und Schwefelatome der an Ringstickstoffatome gebundenen Ketten zusammen mindestens 6 beträgt, oder mindestens 5 beträgt, wenn $R^{2''}$ Isobutyl bedeutet, dadurch gekennzeichnet, daß man ein 4-Hydrazinouracil der allgemeinen Formel III",

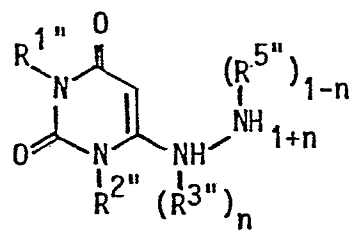

(III"),

worin $R^{1''}$, $R^{2''}$, $R^{3''}$ und $R^{5''}$ die vorstehenden Bedeutungen haben und n die Zahl 1 oder 0 bedeutet, mit einem Aldehyd $R^{4''}$-$C\begin{smallmatrix}{}^{\nearrow O}\\{}_{\searrow H}\end{smallmatrix}$, wobei $R^{4''}$ die vorstehenden Bedeutungen hat, umsetzt und das entstandene Hydrazon dehydrierend cyclisiert bzw. wenn $R^{4''}$ in Formel I" die Bedeutung Wasserstoff hat, das 4-Hydrazinouracil der allgemeinen Formel III" mit Phosphoroxychlorid und Dimethylformamid umsetzt.

15. Verfahren zur Herstellung der Pyrazolo[3,4-d]pyrimidine der allgemeinen Formel I'''

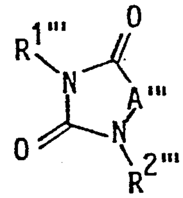

(I'''),

worin einer der Reste $R^{1'''}$ und $R^{2'''}$ für Wasserstoff, Methyl oder Ethyl, der andere für Ethyl, Butyl, Hexyl, 5-Oxo-n-hexyl oder 2-(Dimethylamino)-ethyl, und

A''' für eine Gruppe 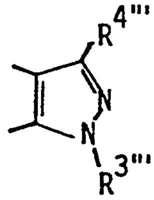 oder 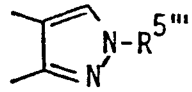

wobei

$R^{3'''}$ Wasserstoff oder Methyl,

$R^{4'''}$ Wasserstoff und

$R^{5'''}$ $C_1$-$C_6$-Alkyl, 2-Hydroxyethyl und Trimethoxybenzoyl bedeuten,

stehen, wobei die Summe der Kohlenstoff-, Sauerstoff- und Stickstoffatome der an Ringstickstoffatome gebundenen Ketten zusammen mindestens 6 beträgt, oder mindestens 5 beträgt, wenn $R^{2'''}$ Isobutyl bedeutet, dadurch gekennzeichnet, daß man ein 4-Hydrazinouracil der allgemeinen Formel III''',

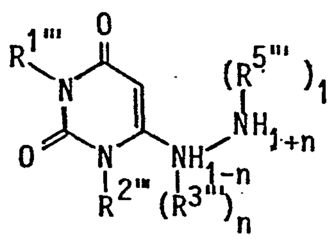 (III'''),

worin $R^{1'''}$, $R^{2'''}$, $R^{3'''}$ und $R^{5'''}$ die vorstehenden Bedeutungen haben und n die Zahl 1 oder 0 bedeutet, mit Phosphoroxychlorid und Dimethylformamid umsetzt.

16. Arzneimittel enthaltend ein oder mehrere Pyrazolo[3,4-d]pyrimidine nach einem oder mehreren der Ansprüche 1, 2, 3, 4, 5, 6, 7, 8, 9, 10 oder 11.

0063381

Europäisches Patentamt

# EUROPÄISCHER RECHERCHENBERICHT

Nummer der Anmeldung

EP 82 10 3302

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,X | JOURNAL OF HETEROCYCLIC CHEMISTRY, Band 15, Nr. 3, Mai 1978, Seiten 359-363, Provo, Utah, USA<br>K. SENGA et al.: "New, convenient synthesis of 2-alkyl- and 2-vinylpyrazolo(3,4-d) pyrimidine derivatives (1)" * Verbindung 3f *<br>--- | 1,16 | C 07 D 487/04<br>A 61 K 31/505//<br>(C 07 D 487/04<br>C 07 D 239/00<br>C 07 D 231/00 ) |
| X | CHEMICAL ABSTRACTS, Band 76, Nr. 25, 19. Juni 1972, Seite 464, Nr. 153707e, Columbus, Ohio, USA<br>S. SENDA et al.: "Pyrimidine derivatives and related compounds. XIII. Synthesis of N-substituted pyrazolo(3,4-d)pyrimidines from pyrazole derivatives"& CHEM. PHARM. BULL. 1972, 20(2), 391-398 * Zusammenfassung *<br>--- | 1,16 | RECHERCHIERTE SACHGEBIETE (Int. Cl. 3) |
| X | DE-A-1 181 711 (ROBUGEN)<br>* Insgesamt *<br>----- | 1,16 | C 07 D 487/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 30-06-1982 | ALFARO I. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82